(19) Europäisches Patentamt / European Patent Office / Office européen des brevets

(11) EP 3 812 365 A1

(12) **EUROPEAN PATENT APPLICATION**
published in accordance with Art. 153(4) EPC

(43) Date of publication:
**28.04.2021 Bulletin 2021/17**

(21) Application number: **19821672.3**

(22) Date of filing: **19.06.2019**

(51) Int Cl.:
***C07C 69/732*** *(2006.01)* ***A23L 33/10*** *(2016.01)*
***A61K 31/216*** *(2006.01)* ***A61P 1/12*** *(2006.01)*
***A61P 13/10*** *(2006.01)* ***A61P 43/00*** *(2006.01)*
***C07C 69/736*** *(2006.01)* ***C07K 14/47*** *(2006.01)*

(86) International application number:
**PCT/JP2019/024322**

(87) International publication number:
**WO 2019/244937 (26.12.2019 Gazette 2019/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **20.06.2018 JP 2018117368**

(71) Applicant: **Kao Corporation**
**Chuo-ku,**
**Tokyo 103-8210 (JP)**

(72) Inventors:
- **TAKAMURA, Akihiro**
  **Haga-gun, Tochigi 321-3497 (JP)**
- **KITAMURA, Naoya**
  **Haga-gun, Tochigi 321-3497 (JP)**
- **YAMAMOTO, Naoki**
  **Haga-gun, Tochigi 321-3497 (JP)**
- **HASHIZUME, Kohjiro**
  **Haga-gun, Tochigi 321-3497 (JP)**

(74) Representative: **Vossius & Partner**
**Patentanwälte Rechtsanwälte mbB**
**Siebertstrasse 3**
**81675 München (DE)**

(54) **ROSMARINIC ACID DERIVATIVE OR SALT THEREOF**

(57) The present invention provides a compound that inhibits the activity of TRPV4 and is useful for prevention or amelioration of an overactive bladder, an irritable bowel syndrome, etc. The compound is a rosmarinic acid derivative represented by the following formula (I) or a salt thereof:

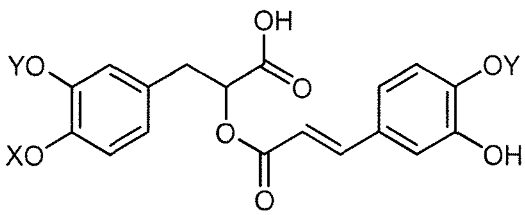

( I )

.

EP 3 812 365 A1

## Description

Field of the Invention

The present invention relates to a rosmarinic acid derivative or a salt thereof that is useful for prevention or amelioration of, for example, an overactive bladder or an irritable bowel syndrome.

Background of the Invention

TRPV4 (transient receptor potential cation channel subfamily V member 4) is one of proteins constituting a thermosensitive TRP channel. TRPV4 is expressed in various tissues, such as the kidney, lung, bladder, heart, skin, brain and digestive tract, and is thought to play various different physiological roles.

TRPV4 is highly expressed in, in particular, the renal distal tubule epithelial cells, and it is suggested that the osmotic pressure and flow rate of urine are sensed by the action of TRPV4 (Non Patent Literature 1). In addition, TRPV4 has been reported to be related to the function of the bladder (Non Patent Literature 2). Specifically, TRPV4 is present in the bladder epithelial cells inside the bladder, and when bladder epithelial cells are stretched by accumulation of urine, calcium flows into the cells through the TRPV channel constituted of TRPV4, etc. This influx of calcium causes the release of ATP from the cells to transmit the bulge of the bladder to nerves. In addition, it has been reported that the influence of TRPV4 on the urination interval is larger than that of other proteins constituting the TRP channel (Non Patent Literature 3).

Even if examination is performed, organic diseases such as inflammation and ulcers are not found on the irritable bowel syndrome (IBS) unlike on enteritis due to bacterial or viral infections and diseases such as ulcerative colitis and colon cancer; but the syndrome is a disease that causes abdominal discomfort, such as diarrhea, constipation, abdominal pain, and tightness of the lower abdomen due to excessive gas. It has been reported that in IBS patients, the amount of a metabolite of polyunsaturated fatty acid (5,6-epoxyeicosatrienoic acid: 5,6-EET), which is a TRPV4 agonist, is large in the colon; and it has also been reported that when the homogenate of the colon containing 5,6-EET is administered to the colon of a mouse, the intestinal mobility is increased to cause exaggerated response of the intestine, but that in this case, the exaggerated response is suppressed by inhibiting the expression of TRPV4 (Non Patent Literature 4).

Accordingly, it is expected to prevent or ameliorate an overactive bladder or irritable bowel syndrome by inhibiting the TRPV4 activity.

On the other hand, it has been reported that specific rosmarinic acid derivatives, such as 3'-O-methyl-rosmarinic acid (i), 4'-O-methyl-rosmarinic acid (ii), or 3,3'-O-dimethyl rosmarinate (iii) represented by the following formulae, have a cyclooxygenase 2 induction-inhibiting activity and have an antiinflammatory effect (Patent Literature 2).

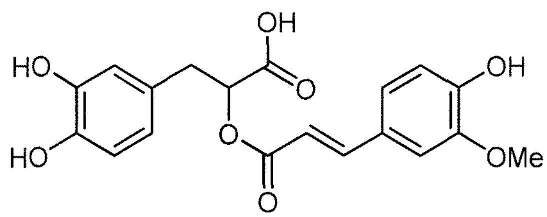

(i)

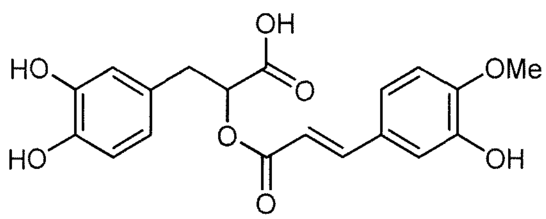

(ii)

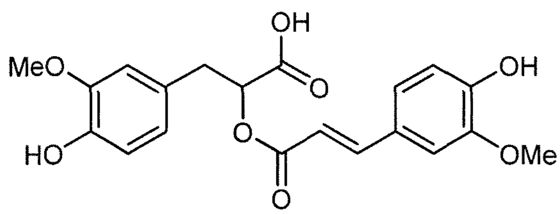

(iii)

Patent Literature

[Patent Literature 1] JP-A-2014-24809
[Patent Literature 2] JP-A-2005-139136

Non Patent Literature

[Non Patent Literature 1] Tian W., Am. J. Physiol. Renal. Physiol., 2004, 287, F17-F24
[Non Patent Literature 2] Nilius B., Physiol. Rev., 2007, 87, 165-217
[Non Patent Literature 3] Gevaert T., J. Clin. Invest., 2007, 117, 3453-3462
[Non Patent Literature 4] Nicolas Cenac et al., Gastroenterology, 2015, 149, 433-444

Summary of the Invention

The present invention relates to the following 1) to 22) :

1) A rosmarinic acid derivative represented by the following formula (I) or a salt thereof;
2) A TRPV4 activity inhibitor comprising a rosmarinic acid derivative represented by the following formula (I) or a salt thereof as an active ingredient;
3) An overactive bladder-preventing or ameliorating agent comprising a rosmarinic acid derivative represented by the following formula (I) or a salt thereof as an active ingredient;
4) An irritable bowel syndrome-preventing or ameliorating agent comprising a rosmarinic acid derivative represented by the following formula (I) or a salt thereof as an active ingredient;
5) A TRPV4 activity-inhibiting food comprising a rosmarinic acid derivative represented by the following formula (I) or a salt thereof as an active ingredient;
6) An overactive bladder-preventing or ameliorating food comprising a rosmarinic acid derivative represented by the following formula (I) or a salt thereof as an active ingredient;
7) An irritable bowel syndrome-preventing or ameliorating food comprising a rosmarinic acid derivative represented by the following formula (I) or a salt thereof as an active ingredient;
8) A use of a rosmarinic acid derivative represented by the following formula (I) or a salt thereof for manufacturing a TRPV4 activity inhibitor;
9) A use of a rosmarinic acid derivative represented by the following formula (I) or a salt thereof for manufacturing an overactive bladder-preventing or ameliorating agent;
10) A use of a rosmarinic acid derivative represented by the following formula (I) or a salt thereof for manufacturing an irritable bowel syndrome-preventing or ameliorating agent;
11) A use of a rosmarinic acid derivative represented by the following formula (I) or a salt thereof for manufacturing a TRPV4 activity-inhibiting food;
12) A use of a rosmarinic acid derivative represented by the following formula (I) or a salt thereof for manufacturing an overactive bladder-preventing or ameliorating food;
13) A use of a rosmarinic acid derivative represented by the following formula (I) or a salt thereof for manufacturing an irritable bowel syndrome-preventing or ameliorating food;
14) A rosmarinic acid derivative represented by the following formula (I) or a salt thereof to for use in inhibiting a TRPV4 activity;
15) A rosmarinic acid derivative represented by the following formula (I) or a salt thereof for use in preventing or ameliorating an overactive bladder;
16) A rosmarinic acid derivative represented by the following formula (I) or a salt thereof for use in preventing or ameliorating an irritable bowel syndrome;
17) A nontherapeutic use of a rosmarinic acid derivative represented by the following formula (I) or a salt thereof for inhibiting a TRPV4 activity;
18) A nontherapeutic use of a rosmarinic acid derivative represented by the following formula (I) or a salt thereof for preventing or ameliorating an overactive bladder;
19) A nontherapeutic use of a rosmarinic acid derivative represented by the following formula (I) or a salt thereof for preventing or ameliorating an irritable bowel syndrome;
20) A method for inhibiting a TRPV4 activity, comprising administering to a subject in need thereof, or allowing a

subject in need thereof to ingest, an effective amount of a rosmarinic acid derivative represented by the following formula (I) or a salt thereof;

21) A method for preventing or ameliorating an overactive bladder, comprising administering to a subject in need thereof, or allowing a subject in need thereof to ingest, an effective amount of a rosmarinic acid derivative represented by the following formula (I) or a salt thereof; and

22) A method for preventing or ameliorating an irritable bowel syndrome, comprising administering to a subject in need thereof, or allowing a subject in need thereof to ingest, an effective amount of a rosmarinic acid derivative represented by the following formula (I) or a salt thereof.

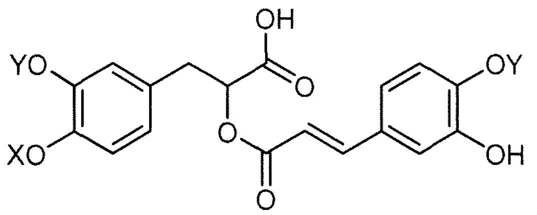

(I)

wherein, X and Y each represent a hydrogen atom or a methyl group; when X is a methyl group, Y represents a hydrogen atom; and when X is a hydrogen atom, Y represents a methyl group.

Detailed Description of the Invention

The present invention relates to provision of a compound that inhibits the activity of TRPV4 and is useful for prevention or amelioration of an overactive bladder, an irritable bowel syndrome, etc.

The present inventors studied rosmarinic acid derivatives and, as a result, found that a rosmarinic acid derivative represented by the following formula (I) has an excellent TRPV4 activity-inhibiting action and is useful for prevention or amelioration of an overactive bladder, an irritable bowel syndrome, etc.

The rosmarinic acid derivative or a salt thereof of the present invention can effectively inhibit the TRPV4 activity and is useful for prevention or amelioration of a disease that is caused by activation of the TRPV4 channel, such as an overactive bladder and an irritable bowel syndrome.

In the rosmarinic acid derivative represented by the formula (I) of the present invention, the following two compounds, 3,4'-di-0-methyl-rosmarinic acid (5) and 4-O-methyl-rosmarinic acid (13), are included. These compounds are novel compounds that have not been isolated or synthesized so far. The compound (5) can also be written as (E)-3-(4-hydroxy-3-methoxyphenyl)-2-{[3-(3-hydroxy-4-methoxyphenyl)acryloyl]oxy}propionic acid, and the compound (13) can also be written as (E)-3-(3-hydroxy-4-methoxyphenyl)-2-{[3-(3,4-dihydroxyphenyl)acryloyl]oxy}propionic acid.

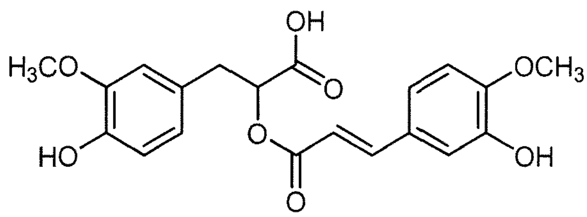

(5)

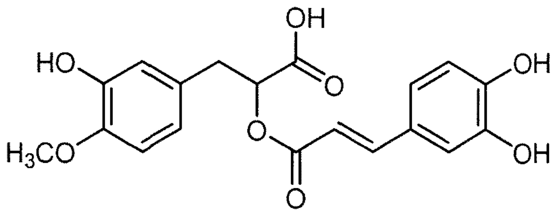

(13)

The rosmarinic acid derivative of the present invention has optical isomers and may be any of the optical isomers or a mixture of the optical isomers. In the present invention, the R-body or a mixture of the optical isomers is preferable.

Examples of the salt of the rosmarinic acid derivative represented by the formula (I) of the present invention include salts with alkali metals such as sodium and potassium, salts with alkaline-earth metals such as calcium and magnesium, ammonium salts, and salts with amines such as triethylamine.

The rosmarinic acid derivative represented by the formula (I) or a salt thereof of the present invention not only can be present in an unsolvated form but also can be present in the form of a hydrate or a solvate. Accordingly, the present invention encompasses all its crystalline forms, hydrates, and solvates. Preferable examples of the solvate include a hydrate, an alcohol solvate, and an acetone solvate.

The rosmarinic acid derivative represented by the formula (I) of the present invention can be synthesized, for example, according to the following reaction scheme, as shown by a manufacturing example, which will be shown later.

(1) Example of synthesis of 3,4'-di-O-methyl-rosmarinic acid (5)

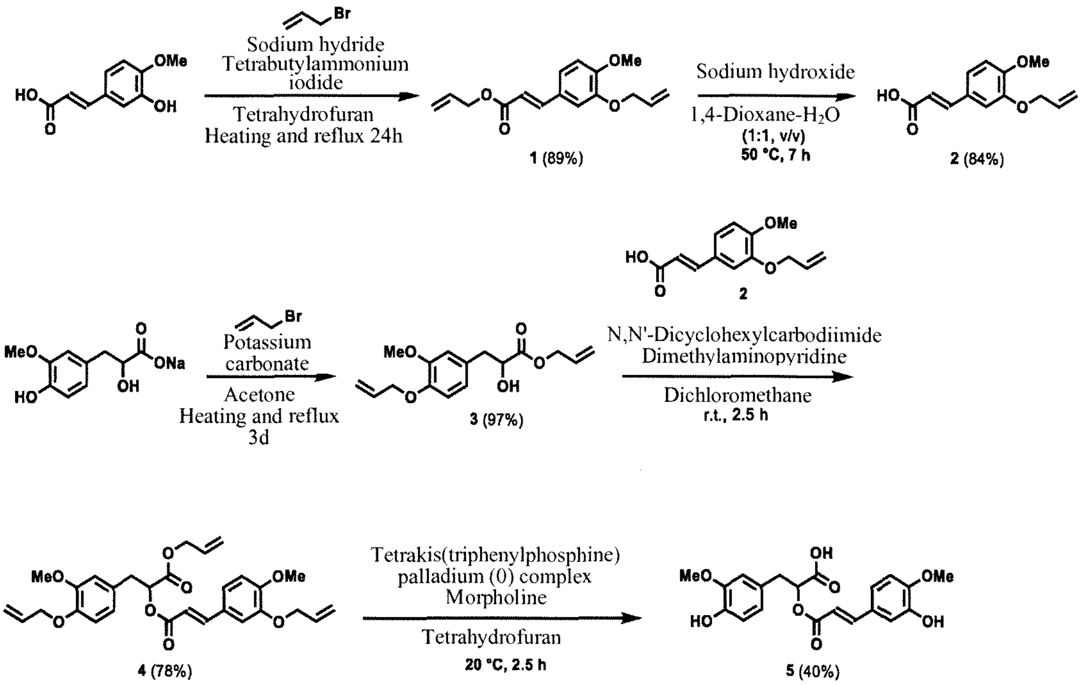


(2) Example of synthesis of 4-O-methyl-rosmarinic acid (13)

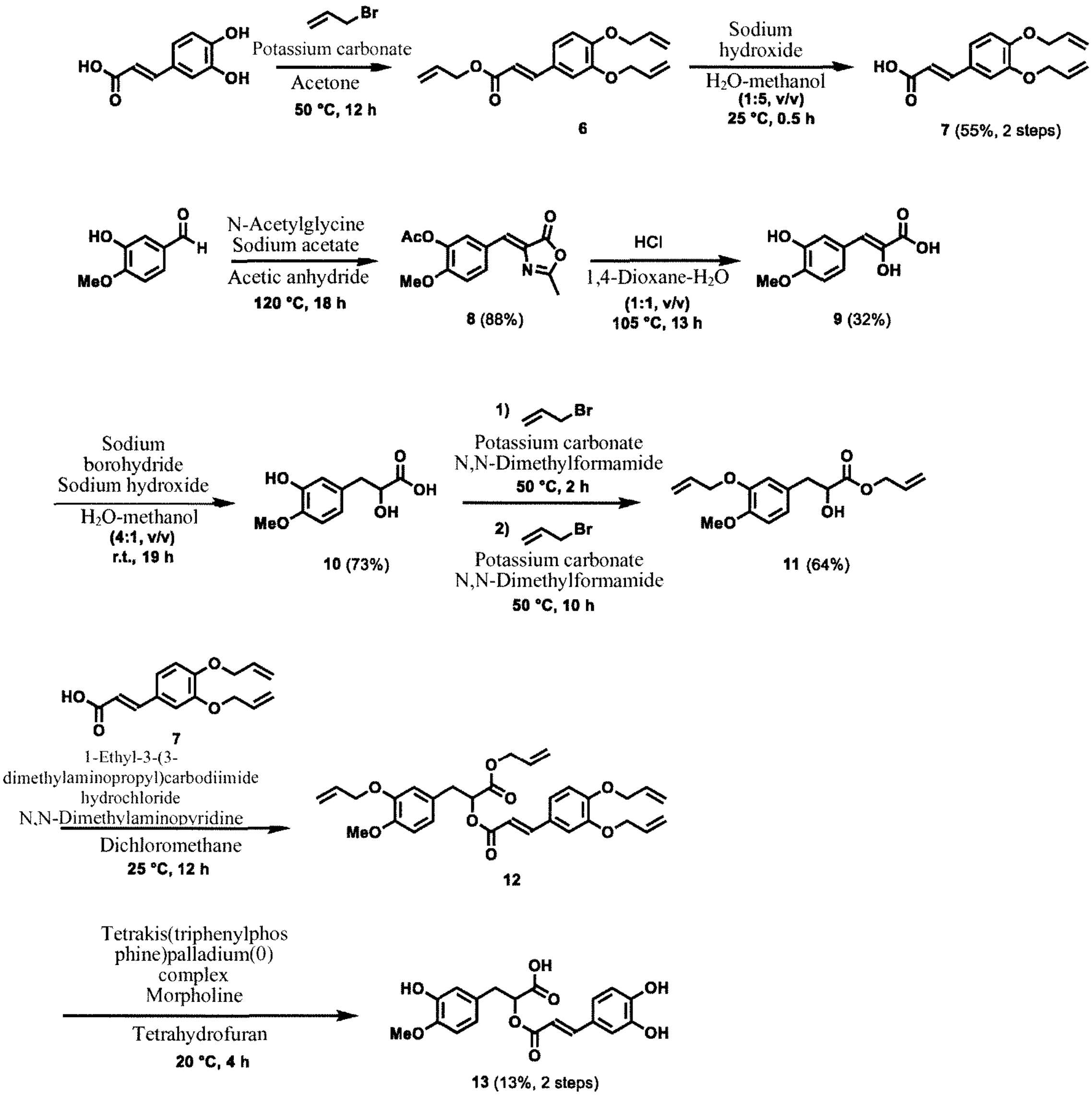


As shown by examples later, the rosmarinic acid derivative represented by the formula (I) inhibits the TRPV4 activity. Specifically, the rosmarinic acid derivative has the following TRPV4 activity-inhibiting action: when a transformant having the TRPV4 gene introduced thereto and thus functionally expressing TRPV4 (TRPV4-expressing cell) is brought into contact with dimethyl rosmarinate in the presence of a TRPV4 stimulator (TRPV4 agonist), the intracellular cation influx by the TRPV4 stimulator is inhibited.

Accordingly, the rosmarinic acid derivative represented by the formula (I) or a salt thereof functions as a TRPV4 activity inhibitor and can be used for inhibiting the TRPV4 activity or for manufacturing a TRPV4 activity inhibitor.

As described above, TRPV4 is present in the bladder epithelial cells inside the bladder. When bladder epithelial cells are stretched by accumulation of urine, calcium is taken into cells through the TRPV4 channel. Consequently, ATP is released from the cell surfaces, and the bulge of the bladder is transmitted to nerves. Accordingly, an overactive bladder can be prevented or ameliorated by inhibiting the activity of TRPV4. In addition, it has been reported that the amount of 5,6-EET (TRPV4 agonist), which is a metabolite of polyunsaturated fatty acid, is large in the colon of irritable bowel syndrome patients; and it has also been reported that when the homogenate of the colon containing 5,6-EET is administered to the colon of a mouse, the intestine exaggeratedly responds, and that the exaggerated response is suppressed by inhibiting the expression of TRPV4 (Non Patent Literature 1). Thus, the irritable bowel syndrome can be prevented or ameliorated by inhibiting the TRPV4 activity.

Accordingly, the rosmarinic acid derivative represented by the formula (I) or a salt thereof functions as an overactive bladder-preventing or ameliorating agent or an irritable bowel syndrome-preventing or ameliorating agent and can be used for preventing or ameliorating an overactive bladder or an irritable bowel syndrome or for manufacturing a preventing or ameliorating agent of an overactive bladder or an irritable bowel syndrome.

In the present invention, the term "TRPV4" means "transient receptor potential cation channel subfamily V member 4". TRPV4 is a protein encoded by a TRPV4 gene in human beings.

The term "TRPV4 activity inhibition" refers to inhibiting the activity of TRPV4 as a receptor and specifically means that an activity expressed by binding of a TRPV4 stimulator to TRPV4, such as ion flux-controlling ability (for example, ability to transport cations, such as calcium ions and sodium ions, from the outside to the inside of cells) or membrane potential-controlling ability (for example, ability to generate electric current), is suppressed or inhibited.

In the present invention, the term "overactive bladder" refers to a symptom syndrome in which urinary urgency is essential and which is usually accompanied by frequent urination or urge urinary incontinence. The term "irritable bowel syndrome" is a generic term for diseases caused mainly by abnormalities of movement and secretory function of the large intestine and means a disease that is diagnosed when a symptom of repeating diarrhea or constipation continues in spite of no abnormalities found by examination, unlike enteritis due to bacterial or viral infections and diseases such as ulcerative colitis and colon cancer.

In the present invention, the "use" for inhibiting the TRPV4 activity or for preventing or ameliorating an overactive bladder or an irritable bowel syndrome can be administration to or ingestion by an animal including a human and may be a therapeutic use or a nontherapeutic use. The term "nontherapeutic" is a concept not including medical practice, specifically, a concept not including methods of surgery, therapy or diagnosis of humans, more specifically, a concept not including methods of surgery, therapy or diagnosis of humans that are practiced by medical doctors or those who are directed by medical doctors.

In the present specification, the term "prevention" refers to preventing or delaying the onset of a disease or a symptom in an individual or reducing the risk of developing a disease or a symptom in an individual. The term of "amelioration" refers to recovery from a disease, symptom or condition, prevention or delay of deterioration of a disease, symptom or condition, or reversal, prevention or delay of progression of a disease, symptom or condition.

The TRPV4 activity inhibitor or the overactive bladder or irritable bowel syndrome-preventing or ameliorating agent of the present invention can be a drug, a quasi-drug, a supplement or a food exhibiting a TRPV4 activity-inhibiting effect or the effect of preventing or ameliorating overactive bladder or irritable bowel syndrome when ingested by or administered to an animal including a human, or can be a material or preparation to be blended therein.

Examples of the food of the present invention include general foods and drinks, and also foods claiming uses as needed, functional foods, foods for patients, special health foods, foods with functional claims, and supplements.

The above-mentioned drugs (including quasi-drugs) containing the rosmarinic acid derivative represented by the formula (I) or a salt thereof of the present invention can be administered in any dosage form and are preferably orally administered. In the administration, the active ingredient is mixed with a solid or liquid medical nontoxic carrier suitable for an administration method such as oral administration, intrarectal administration or injection and can be administered in a common pharmaceutical preparation form.

Examples of these preparations include solid agents, such as tablets, granules, powders and capsules; liquid agents, such as solution, suspension and emulsion; and lyophilized agents. These preparations can be prepared by usual procedures for formulation and can contain common additives, such as a stabilizer, a wetting agent, an emulsifier, a binder, a tonicity agent and an excipient, as needed.

Examples of the form of the food containing the rosmarinic acid derivative represented by the formula (I) or a salt thereof of the present invention include various foods, such as foods and drinks and nutritional foods, for example, soft drinks, tea drinks, coffee drinks, fruit juice drinks, carbonated drinks, jellies, wafers, biscuits, bread, noodles and sausages, and further include nutrition support compositions in the same forms as those described for the above-mentioned oral administration preparations (solid preparations such as tablets, capsules and lozenges). In particular, tablets are preferable, and chewable tablets are more preferable.

In order to prepare foods in a variety of forms, the rosmarinic acid derivative represented by the formula (I) or a salt thereof of the present invention can be used alone or can be used in an appropriate combination with another food material or a solvent, a softener, an oil, an emulsifier, a preservative, a flavoring agent, a stabilizer, a coloring agent, an antioxidant, a moisturizer, a thickener, or the like.

The content of the rosmarinic acid derivative represented by the formula (I) or a salt thereof of the present invention in the above-mentioned drugs (including quasi-drugs) and foods varies depending on the usage thereof and, usually, is preferably 0.01 mass% or more, more preferably 0.1 mass% or more, and even more preferably 1 mass% or more and preferably 100 mass% or less, more preferably 90 mass% or less, and even more preferably 70 mass% or less. In addition, the content is preferably 0.01 to 100 mass%, more preferably 0.1 to 90 mass%, and even more preferably 1 to 70 mass%.

When the rosmarinic acid derivative represented by the formula (I) or a salt thereof of the present invention is used as a drug or food or blended into a drug or food before use, the dosage for a human varies depending on the condition, weight, sex, and age of the subject or another factor, and in the case of oral administration, the daily dose per adult is usually preferably 0.1 mg or more, more preferably 1 mg or more, and even more preferably 10 mg or more and preferably 2,000 mg or less, more preferably 500 mg or less, and even more preferably 200 mg or less. In addition,

the dose is preferably 0.1 to 2,000 mg, more preferably 1 to 500 mg, and even more preferably 10 to 200 mg, in terms of a rosmarinic acid derivative represented by the formula (I) or a salt thereof.

In addition, the above-mentioned preparation can be administered according to an arbitrary administration schedule and is preferably administered once to several times a day for several weeks to several months continuously.

The subject for administration or ingestion is not particularly limited as long as it is an animal including a human who needs or wishes to inhibit the TRPV4 activity or to prevent or ameliorate an overactive bladder or an irritable bowel syndrome; however, it is effective to administrate to or be ingested by humans having symptoms, for example, an overactive bladder or urinary disturbance such as urinary urgency and urge urinary incontinence or humans having symptoms such as diarrhea or constipation due to stress, abdominal pain, or tightness of the lower abdomen due to excessive gas.

Regarding the above-described embodiments, the present invention discloses the following aspects:

<1> A rosmarinic acid derivative represented by the following formula (I) or a salt thereof;

<2> A TRPV4 activity inhibitor comprising a rosmarinic acid derivative represented by the following formula (I) or a salt thereof as an active ingredient;

<3> An overactive bladder-preventing or ameliorating agent comprising a rosmarinic acid derivative represented by the following formula (I) or a salt thereof as an active ingredient;

<4> An irritable bowel syndrome-preventing or ameliorating agent comprising a rosmarinic acid derivative represented by the following formula (I) or a salt thereof as an active ingredient;

<5> A TRPV4 activity-inhibiting food comprising a rosmarinic acid derivative represented by the following formula (I) or a salt thereof as an active ingredient;

<6> An overactive bladder-preventing or ameliorating food comprising a rosmarinic acid derivative represented by the following formula (I) or a salt thereof as an active ingredient;

<7> An irritable bowel syndrome-preventing or ameliorating food comprising a rosmarinic acid derivative represented by the following formula (I) or a salt thereof as an active ingredient;

<8> A use of a rosmarinic acid derivative represented by the following formula (I) or a salt thereof for manufacturing a TRPV4 activity inhibitor;

<9> A use of a rosmarinic acid derivative represented by the following formula (I) or a salt thereof for manufacturing an overactive bladder-preventing or ameliorating agent;

<10> A use of a rosmarinic acid derivative represented by the following formula (I) or a salt thereof for manufacturing an irritable bowel syndrome-preventing or ameliorating agent;

<11> A use of a rosmarinic acid derivative represented by the following formula (I) or a salt thereof for manufacturing a TRPV4 activity-inhibiting food;

<12> A use of a rosmarinic acid derivative represented by the following formula (I) or a salt thereof for manufacturing an overactive bladder-preventing or ameliorating food;

<13> A use of a rosmarinic acid derivative represented by the following formula (I) or a salt thereof for manufacturing an irritable bowel syndrome-preventing or ameliorating food;

<14> A rosmarinic acid derivative represented by the following formula (I) or a salt thereof for use in inhibiting a TRPV4 activity;

<15> A rosmarinic acid derivative represented by the following formula (I) or a salt thereof for use in preventing or ameliorating an overactive bladder;

<16> A rosmarinic acid derivative represented by the following formula (I) or a salt thereof for use in preventing or ameliorating an irritable bowel syndrome;

<17> A nontherapeutic use of a rosmarinic acid derivative represented by the following formula (I) or a salt thereof for inhibiting a TRPV4 activity;

<18> A nontherapeutic use of a rosmarinic acid derivative represented by the following formula (I) or a salt thereof for preventing or ameliorating an overactive bladder;

<19> A nontherapeutic use of a rosmarinic acid derivative represented by the following formula (I) or a salt thereof for preventing or ameliorating an irritable bowel syndrome;

<20> A method for inhibiting a TRPV4 activity, comprising administering to a subject in need thereof, or allowing a subject in need thereof to ingest, an effective amount of a rosmarinic acid derivative represented by the following formula (I) or a salt thereof;

<21> A method for preventing or ameliorating an overactive bladder, comprising administering to a subject in need thereof, or allowing a subject in need thereof to ingest, an effective amount of a rosmarinic acid derivative represented by the following formula (I) or a salt thereof; and

<22> A method for preventing or ameliorating an irritable bowel syndrome, comprising administering to a subject in need thereof, or allowing a subject in need thereof to ingest, an effective amount of a rosmarinic acid derivative represented by the following formula (I) or a salt thereof.

<23> In each of the TRPV4 activity inhibitor according to <2> or <8>, the overactive bladder-preventing or ameliorating agent according to <3> or <9>, the irritable bowel syndrome-preventing or ameliorating agent according to <4> or <10>, the TRPV4 activity-inhibiting food according to <5> or <11>, the overactive bladder-preventing or ameliorating food according to <6> or <12>, and the irritable bowel syndrome-preventing or ameliorating food according to <7> or <13>, the content of the active ingredient is preferably 0.01 mass% or more, more preferably 0.1 mass% or more, and even more preferably 1 mass% or more and preferably 100 mass% or less, more preferably 90 mass% or less, and even more preferably 70 mass% or less and preferably 0.01 to 100 mass%, more preferably 0.1 to 90 mass%, and even more preferably 1 to 70 mass%, based on the total amount.

<24> In <14> to <23>, the daily dose per adult is preferably 0.1 mg or more, more preferably 1 mg or more, and even more preferably 10 mg or more and preferably 2,000 mg or less, more preferably 500 mg or less, and even more preferably 200 mg or less and preferably 0.1 to 2,000 mg, more preferably 1 to 500 mg, and even more preferably 10 to 200 mg, in terms of the rosmarinic acid derivative represented by the formula (I) or a salt thereof.

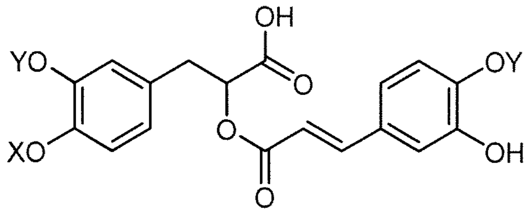

(I)

wherein, X and Y each represent a hydrogen atom or a methyl group; when X is a methyl group, Y represents a hydrogen atom; and when X is a hydrogen atom, Y represents a methyl group.

Examples

The present invention will now be described in more detail by way of examples but is not limited thereto. Manufacturing Example 1: Synthesis of 3,4'-di-O-methyl-rosmarinic acid and 4-O-methyl-rosmarinic acid

<Reagent>

Isoferulic acid, sodium 3-(4-hydroxy-3-methoxyphenyl)lactate, isovanillin, caffeic acid, and N-acetylglycine were purchased from Tokyo Chemical Industry Co., Ltd.; ferulic acid and sodium acetate were purchased from Sigma-Aldrich Co. LLC.; allyl bromide, N,N'-dicyclohexylcarbodiimide (DCC), N,N-dimethylaminopyridine (DMAP), morpholine, super dehydrated acetone, super dehydrated N,N-dimethylformamide (DMF), super dehydrated tetrahydrofuran, super dehydrated 1,4-dioxane, super dehydrated dichloromethane, 55% sodium hydride, tetrabutylammonium iodide (TBAI), 1-ethyl-3-(3-dimethylaminopropyl)carbodiimide hydrochloride (EDCI), tetrakis(triphenylphosphine)palladium(0) complex [$Pd(PPh_3)_4$], 8 N sodium hydroxide aqueous solution, anhydrous sodium sulfate, and tert-butyl methyl ether were purchased from FUJIFILM Wako Pure Chemical Corporation; and potassium carbonate, dichloromethane, n-hexane, ethyl acetate, methanol, chloroform, acetone, concentrated hydrochloric acid, formic acid, acetic acid, and sodium borohydride were purchased from Kanto Chemical Co., Inc.

1) Synthesis of allyl (E)-3-[4-(allyloxy)-3-methoxyphenyl]acrylate (1)

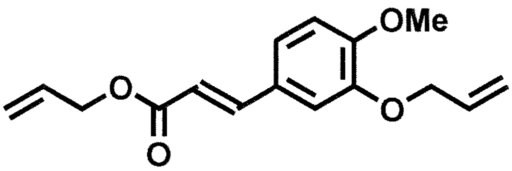

1

In an argon atmosphere, super dehydrated tetrahydrofuran (7 mL) was added to isoferulic acid (1.50 g, 7.72 mmol). Furthermore, 55% sodium hydride (1.01 g, 23.2 mmol), allyl bromide (16.8 mL, 139 mmol), and TBAI (285 mg, 0.77 mmol) were sequentially added thereto, followed by stirring for 24 hours under reflux and heating at 70°C. Subsequently, 8 N sodium hydroxide aqueous solution (16 mL) and deionized water (100 mL) were added to the reaction system, followed by extraction with chloroform (100 mL) three times. The organic layer was dried over anhydrous sodium

sulfate, and the solvent was then distilled away under reduced pressure. The resulting residue was purified by silica gel column chromatography (eluent: n-hexane-ethyl acetate (85:15, v/v)) to obtain 1.88 g (89%) of the target compound.

Rf on the normal phase TLC of the target compound = [migration distance of target compound spot]/[migration distance of solvent front] = 0.71 (n-hexane-ethyl acetate-acetic acid, 50:50:1, v/v/v)
$^1$H NMR (600 MHz, chloroform-d) δ 7.64 (d, J = 15.9 Hz, 1H), 7.11 (dd, J = 8.3, 2.1 Hz, 1H), 7.06 (d, J = 1.7 Hz, 1H), 6.87 (d, J = 8.5 Hz, 1H), 6.30 (d, J = 15.8 Hz, 1H), 6.09 (ddd, J = 22.6, 10.8, 5.3 Hz, 1H), 6.00 (ddd, J = 22.7, 10.8, 5.5 Hz, 1H), 5.40 (dddd, J = 31.2, 17.1, 2.8, 1.7 Hz, 2H), 5.30 (dddd, J = 27.0, 10.3, 2.5, 1.1 Hz, 2H), 4.71 (dt, J = 5.5, 1.3 Hz, 1H), 4.64 (dt, J = 5.3, 1.4 Hz, 1H), 3.91 (s, 3H).

2) Synthesis of (E)-3-[4-(allyloxy)-3-methoxyphenyl]acrylic acid (2)

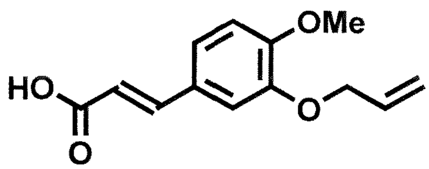

2

In an argon atmosphere, super dehydrated 1,4-dioxane (34 mL) and 0.4 N sodium hydroxide aqueous solution (34 mL) were added to the compound (1) (1.88 g, 6.86 mmol), followed by stirring at 50°C for 7 hours. Subsequently, deionized water (100 mL) was added to the reaction system, followed by washing with chloroform (100 mL) twice. Furthermore, 1.5 N hydrochloric acid aqueous solution (10 mL) was added to the aqueous layer, followed by extraction with chloroform (100 mL) three times. The organic layer was dried over anhydrous sodium sulfate, and the solvent was then distilled away under reduced pressure to obtain 1.40 g (84%) of the target compound.

Rf on the normal phase TLC of the target compound = 0.30 (n-hexane-ethyl acetate-acetic acid, 50:50:1, v/v/v)
$^1$H NMR (600 MHz, chloroform-d) δ 7.71 (d, J = 15.8 Hz, 1H), 7.14 (dd, J = 8.3, 1.9 Hz, 1H), 7.09 (d, J = 1.9 Hz, 1H), 6.89 (d, J = 8.3 Hz, 1H), 6.29 (d, J = 15.8 Hz, 1H), 6.09 (ddd, J = 22.6, 10.5, 5.3 Hz, 1H), 5.44 (ddd, J = 17.3, 3.0, 1.5 Hz, 1H), 5.33 (ddd, J = 10.5, 2.6, 1.1 Hz, 1H), 4.65 (dt, J = 5.7, 1.6 Hz, 2H), 3.92 (s, 3H).

3) Synthesis of allyl 3-[4-(allyloxy)-3-methoxyphenyl]-2-hydroxypropionate (3)

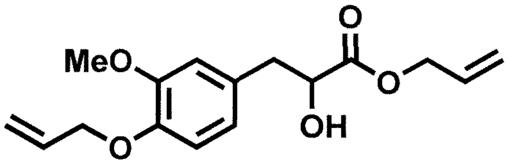

3

In an argon atmosphere, super dehydrated acetone (12.6 mL) was added to sodium 3-(4-hydroxy-3-methoxyphenyl) lactate (1.0 g, 4.27 mmol) and potassium carbonate (1.77 g, 12.8 mmol). Furthermore, allyl bromide (5.41 mL, 64.1 mmol) was added thereto, followed by stirring for 3 days under reflux and heating at 60°C. Subsequently, ethyl acetate (100 mL) was added to the reaction system, followed by washing with deionized water (100 mL) twice. The organic layer was dried over anhydrous sodium sulfate, and the solvent was then distilled away under reduced pressure. The resulting residue was purified by silica gel column chromatography (eluent: n-hexane-ethyl acetate (60:40, v/v)) to obtain 1.21 g (97%) of the target compound.

Rf on the normal phase TLC of the target compound = 0.64 (n-hexane-ethyl acetate-acetic acid, 50:50:1, v/v/v)
$^1$H NMR (600 MHz, chloroform-d) δ 6.80 (d, J = 8.3 Hz, 1H), 6.76 (d, J = 2.5 Hz, 1H), 6.72 (dd, J = 7.9, 1.9 Hz, 1H), 6.12-6.06 (m, 1H), 6.03-5.96 (m, 1H), 5.44-5.36 (m, 2H), 5.33-5.26 (m, 2H), 5.27 (ddd, J = 10.5, 2.6, 1.5 Hz, 1H), 4.66 (dt, J = 6.0, 1.3 Hz, 2H), 4.59 (dt, J = 5.8, 1.6 Hz, 2H), 4.45 (dd, J = 6.2, 4.6 Hz, 1H), 3.85 (s, 3H), 3.08 (dd, J = 13.8, 4.7 Hz, 1H), 2.93 (dd, J = 14.1, 6.6 Hz, 1H).

4) Synthesis of methyl 3',4-di-O-allyl-3,4'-di-O-methyl-rosmarinate (4)

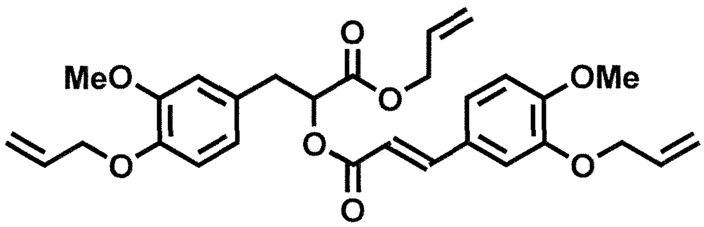

4

In an argon atmosphere, super dehydrated dichloromethane (1.05 mL) was added to the compound (3) (61.4 mg, 0.21 mmol). Furthermore, the compound (2) (66.4 mg, 0.27 mmol) and DMAP (77.0 mg, 0.63 mmol) were sequentially added thereto. The reaction system was cooled to -10°C, and DCC (65.0 mg, 0.32 mmol) was then added to the reaction system, followed by stirring at room temperature for 2.5 hours. Subsequently, the reaction solution was filtered, and the filtration residue was washed with chloroform (3 mL). The solvent of the filtrate was distilled away under reduced pressure, and the resulting residue was purified by silica gel column chromatography (eluent: n-hexane-ethyl acetate (50:50, v/v)) to obtain 106 mg (78%) of the target compound.

Rf on the normal phase TLC of the target compound = 0.65 (n-hexane-ethyl acetate-acetic acid, 50:50:1, v/v/v) $^{1}$H NMR (600 MHz, chloroform-d) δ 7.63 (d, J = 16.2 Hz, 1H), 7.09 (dd, J = 8.3, 1.8 Hz, 1H), 7.05 (d, J = 2.3 Hz, 1H), 6.87 (d, J = 8.3 Hz, 1H), 6.81 (d, J = 7.9 Hz, 1H), 6.79 (d, J = 1.8 Hz, 1H), 6.78 (dd, J = 8.0, 1.9 Hz, 1H), 6.30 (d, J = 15.8 Hz, 1H), 6.12-6.04 (m, 2H), 5.86 (ddd, J = 22.9, 10.4, 5.8 Hz, 1H), 5.45-5.22 (m, 6H), 4.64 (dd, J = 5.3, 1.5 Hz, 2H), 3.91 (s, 3H), 3.85 (s, 3H), 3.19 (dd, J = 14.5, 4.7 Hz, 1H), 3.14 (dd, J = 14.3, 8.3 Hz, 1H).

5) Synthesis of 3,4'-di-O-methyl-rosmarinic acid (5)

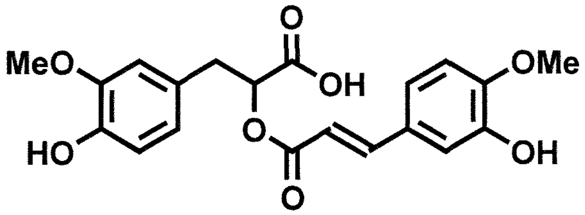

5

In an argon atmosphere, super dehydrated tetrahydrofuran (2.5 mL), $Pd(PPh_3)_4$ (5.7 mg, 5.0 μmol), and morpholine (256 μL, 3.0 mmol) were sequentially added to the compound (4) (49.5 mg, 0.10 mmol), followed by stirring at room temperature. $Pd(PPh_3)_4$ (11.4 mg, 9.9 μmol) was added to the reaction system every 15 minutes from the start of the reaction, 0.09 mmol in total, and stirring was performed for 2.5 hours in total. Subsequently, deionized water (40 mL) and 1.5 N hydrochloric acid aqueous solution (2.9 mL) were added to the reaction system, followed by extraction with ethyl acetate (40 mL) twice. The organic layer was dried over anhydrous sodium sulfate, and the solvent was then distilled away under reduced pressure. The resulting residue was purified by silica gel column chromatography (eluent: chloroform-methanol (96:4, v/v)) to obtain 15.2 mg (40%) of the target compound.

Rf on the normal phase TLC of the target compound = 0.19 (chloroform-acetone-formic acid, 75:20:5, v/v/v)
$^{1}$H NMR (600 MHz, methanol-d) δ 7.58 (d, J = 15.9 Hz, 1H), 7.07 (d, J = 2.0 Hz, 1H), 7.05 (dd, J = 5.5, 2.1 Hz, 1H), 6.97 (d, J = 8.3 Hz, 1H), 6.89 (d, J = 1.4 Hz, 1H), 6.74 (dd, J = 5.4, 1.6 Hz, 1H), 6.72 (d, J = 7.9 Hz, 1H), 6.33 (d, J = 15.9 Hz, 1H), 5.21 (dd, J = 5.7, 3.4 Hz, 1H), 3.89 (s, 3H), 3.83 (s, 3H), 3.20-3.07 (m, 1H), 3.07 (dd, J = 9.6, 8.9 Hz, 1H).
$^{13}$C NMR (600 MHz, methanol-d) δ 172.5, 166.7, 149.1, 146.4, 145.8, 145.7, 144.3, 127.9, 127.2, 121.8, 121.7, 114.5, 114.2, 113.0.111.9, 110.5, 67.2, 55.6, 55.5, 36.8.
ESI-MS calcd for $C_{20}H_{19}O_8^-$ $[M-H]^-$ 387.1; found 387.0.

6) Synthesis of allyl (E)-3-[3,4-bis(allyloxy)phenyl]acrylate (6)

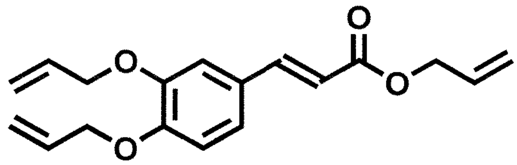

6

Super dehydrated acetone (10 mL), potassium carbonate (2.30 mg, 16.6 mmol), and allyl bromide (4.03 g, 33.3 mmol) were sequentially added to caffeic acid (1.00 g, 5.55 mmol), followed by stirring at 50°C for 12 hours. Subsequently, deionized water (30 mL) was added thereto, and acetone was distilled away under reduced pressure. The aqueous layer was extracted with ethyl acetate (20 mL) three times. The organic layer was dried over anhydrous sodium sulfate, and the solvent was then distilled away under reduced pressure. The resulting target compound (1.50 g (crude)) as a crudely purified product was directly used in the subsequent reaction.

7) Synthesis of (E)-3-[3,4-bis(allyloxy)phenyl]acrylic acid (7)

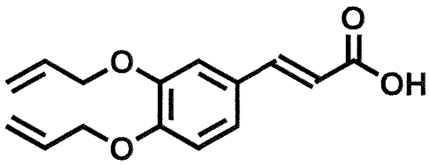

7

The compound (6) (1.50 g, 4.99 mmol) was dissolved in methanol (10 mL). Furthermore, 2 mL of deionized water in which sodium hydroxide (599 mg, 14.9 mmol) was dissolved was added thereto, followed by stirring at 25°C for 30 minutes. Subsequently, 1 N hydrochloric acid aqueous solution (20 mL) was added thereto, followed by extraction with ethyl acetate (20 mL) three times. The organic layer was dried over anhydrous sodium sulfate. Methyl tert-butyl methyl ether (5 mL) was added to the resulting residue, followed by stirring at 25°C for 1 hour and then filtration. The filter residue was washed with tert-butyl methyl ether (5 mL) three times to obtain 0.80 g (60% (2 steps)) of the target compound.

Rf on the normal phase TLC of the target compound = 0.55 (chloroform-methanol-formic acid, 95:5:1, v/v/v) $^1$H NMR (400 MHz, DMSO-$d_6$) δ 12.20 (s, 1H), 7.50 (d, J = 16.0 Hz, 1H), 7.33 (s, 1H), 7.18 (d, J = 1.6 Hz, 1H), 6.99 (d, J = 8.4 Hz, 1H), 6.42 (d, J = 16.0 Hz, 1H), 6.07-6.02 (m, 2H), 5.44 (d, J = 1.6 Hz, 1H), 5.43 (d, J = 1.6 Hz, 1H), 5.26 (d, J = 10.4 Hz, 2H), 4.63-4.60 (m, 4H).

8) Synthesis of (4Z)-4-{[3-(acetyloxy)-4-methoxyphenyl]methylene}-2-methyl-5(4H)-oxazolone (8)

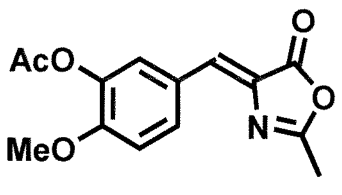

8

The target compound (32.5 g (88%)) was synthesized by the method described in Patent Literature (KR 2013087877) using isovanillin as a starting material.

9) Synthesis of (Z)-2-hydroxy-3-(3-hydroxy-4-methoxyphenyl)acrylic acid (9)

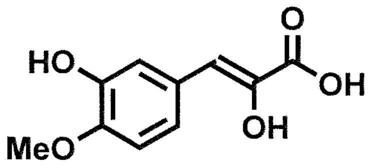

9

Super dehydrated 1,4-dioxane (64 mL) and 1 N hydrochloric acid aqueous solution (64 mL) were added to the compound (8) (5.0 g, 21.4 mmol), followed by stirring at 105°C for 13 hours. Subsequently, deionized water (100 mL) was added to the reaction system, followed by extraction with ethyl acetate (150 mL) twice. The organic layer was dried over anhydrous sodium sulfate, and the solvent was then distilled away under reduced pressure. Chloroform (50 mL) was added to the resulting residue for suspending the residue, followed by filtration. The filter residue was washed with chloroform (10 mL) twice. The resulting residue was subjected to solid-liquid extraction using methanol (70 mL). The solvent of the collected filtrate was distilled away under reduced pressure to obtain 1.45 g (32%) of the target compound.

Rf on the normal phase TLC of the target compound = 0.42 (chloroform-methanol-formic acid, 70:30:1, v/v/v) $^1$H NMR (600 MHz, DMSO-$d_6$) $\delta$ 12.98 (br, 1H), 8.96 (br, 1H), 8.91 (br, 1H), 7.35 (d, J = 2.2 Hz, 1H), 7.06 (dd, J = 8.5, 2.1 Hz, 1H), 6.87 (d, J = 8.5 Hz, 1H), 6.26 (s, 1H), 3.75 (s, 3H).

10) Synthesis of 3-(3-hydroxy-4-methoxyphenyl)lactic acid (10)

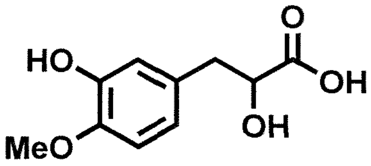

10

Methanol (0.8 mL) and deionized water (2.4 mL) were added to the compound (9) (300 mg, 1.43 mmol). The reaction system was ice-cooled, and 1 N sodium hydroxide aqueous solution (1.5 mL) was added to the reaction system to adjust the pH to 10, and sodium borohydride (81 mg, 2.15 mmol) was added thereto. The reaction system was warmed to room temperature, followed by stirring for 19 hours. Subsequently, under ice cooling, 1.5 N hydrochloric acid aqueous solution was dropwise added to the reaction system to adjust the pH to 2. Furthermore, deionized water (100 mL) was added thereto, followed by extraction with ethyl acetate (70 mL) three times. The organic layer was dried over anhydrous sodium sulfate, and the solvent was then distilled away under reduced pressure. Methanol (10 mL) and silica gel (1.5 g) were added to the resulting residue, and the solvent was distilled away under reduced pressure. The resulting residue was purified by silica gel column chromatography (eluent: 1% formic acid-containing chloroform-methanol (98:2, v/v)) to obtain 221 mg (73%) of the target compound.

Rf on the normal phase TLC of the target compound = 0.63 (chloroform-methanol-formic acid, 70:30:1, v/v/v) $^1$H NMR (400 MHz, methanol-d) $\delta$ 6.81 (d, J = 8.0 Hz, 1H), 6.76 (d, J = 1.6 Hz, 1H), 6.69 (dd, J = 8.4, 1.6 Hz, 1H), 4.29 (ddd, J = 8.0, 4.4, 1.2 Hz, 1H), 3.80 (s, 3H), 2.98 (dd, J = 14.0, 4.4 Hz, 1H), 2.98 (dd, J = 14.0, 4.6 Hz, 1H).

11) synthesis of allyl 3-(3-(allyloxy)-4-methoxyphenyl)-2-hydroxypropionate (11)

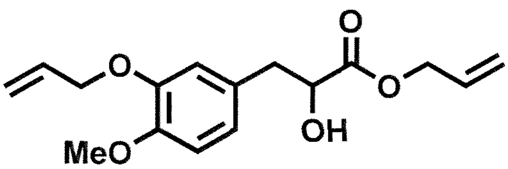

11

The compound (10) (660 mg, 2.82 mmol) was put in a 50-mL round-bottom flask, super dehydrated DMF (10 mL), potassium carbonate (779 mg, 5.64 mmol), and allyl bromide (1.70 g, 14.0 mmol) were sequentially added to the flask, followed by stirring at 50°C for 2 hours. Subsequently, deionized water (20 mL) was added thereto, followed by extraction with ethyl acetate (20 mL) three times. The collected organic layer was washed with deionized water (20 mL)

three times and dried over anhydrous sodium sulfate, and the solvent was then distilled away under reduced pressure. The resulting residue was dissolved in super dehydrated DMF (10 mL), and potassium carbonate (779 mg, 5.64 mmol) and allyl bromide (340 mg, 2.82 mmol) were then subsequently added thereto, followed by stirring at 50°C for 10 hours. Subsequently, deionized water (20 mL) was added thereto, followed by extraction with ethyl acetate (20 mL) three times. The collected organic layer was washed with deionized water (20 mL) three times and dried over anhydrous sodium sulfate, and the solvent was then distilled away under reduced pressure. The resulting target compound (530 mg (crude)) as a crudely purified product was directly used in the subsequent reaction.

12) Synthesis of allyl 3,3',4'-tri-O-allyl-4-O-methyl-rosmarinate (12)

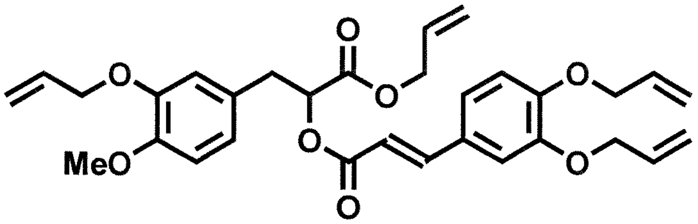

12

The compound (11) (600 mg, 2.05 mmol) and the compound (7) (534 mg, 2.05 mmol) were dissolved in super dehydrated dichloromethane (5 mL), and DMAP (25.0 mg, 205 μmol) and EDCI (393 mg, 2.05 mmol) were added thereto, followed by stirring at 25°C for 10 hours. Subsequently, the reaction system was washed with saturated sodium hydrogen carbonate aqueous solution (15 mL) three times and with 1% citric acid aqueous solution (15 mL) three times. The organic layer was dried over anhydrous sodium sulfate, and the solvent was distilled away under reduced pressure. The resulting target compound (600 mg (crude)) as a crudely purified product was directly used in the subsequent reaction.

13) Synthesis of 4-O-methyl-rosmarinic acid (13)

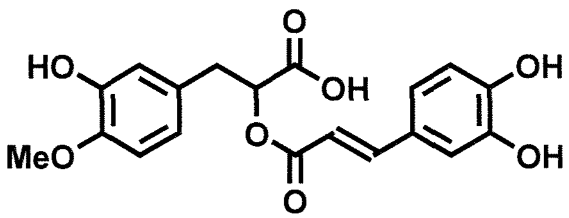

13

The compound (12) (600 mg, 1.12 mmol) was dissolved in super dehydrated tetrahydrofuran (10 mL), and $Pd(PPh_3)_4$ (389 mg, 336 μmol) and morpholine (977 mg, 11.2 mmol) were added thereto, followed by stirring at 25°C for 4 hours. Subsequently, deionized water (100 mL) was added thereto, and the pH was adjusted to 5 by dropwise addition of 1 N hydrochloric acid aqueous solution. Furthermore, extraction with ethyl acetate (30 mL) was performed three times, and the collected organic layer was washed with saturated sodium hydrogen carbonate aqueous solution (30 mL) three times. The pH of the collected aqueous layer was adjusted to 5 by dropwise addition of 1 N hydrochloric acid aqueous solution, followed by extraction with ethyl acetate (30 mL) three times. All organic layers were collected and dried over anhydrous sodium sulfate, and the solvent was distilled away under reduced pressure. The resulting residue was purified by silica gel column chromatography (eluent: 0.1% formic acid aqueous solution-acetonitrile (75:25, v/v)) to obtain 100.5 mg (24% (2 steps)) of the target compound.

Rf on the normal phase TLC of the target compound = 0.39 (chloroform-methanol-formic acid, 90:10:1, v/v/v)
$^1H$ NMR (400 MHz, DMSO-$d_6$) δ 13.1 (s, 1H), 9.67 (s, 1H), 9.18 (s, 1H), 8.90 (s, 1H), 7.46 (d, J = 15.6 Hz, 1H), 7.06 (d, J = 2 Hz, 1H), 7.03 (dd, J = 8.0, 2.0 Hz, 1H), 6.82 (d, J = 8.0 Hz, 1H), 6.77 (d, J = 8.0 Hz, 1H), 6.77 (d, J = 2.0 Hz, 1H), 6.66 (dd, J = 10.4 Hz, 1H), 6.24 (d, J = 16.0 Hz, 1H), 5.05 (dd, 8.4, 4.4 Hz, 1H), 3.72 (s, 3H), 3.03 (dd, 14.4, 4.4, 1H), 2.94 (dd, 14.0, 8.4 Hz, 1H).
$^{13}C$ NMR (600 MHz, methanol-d) δ 173.4, 168.4, 149.8, 148.1, 147.7, 147.4, 146.8, 130.6, 127.6, 123.2, 121.7, 117.5, 116.5, 115.2, 114.3, 112.7, 74.5, 56.4, 37.9.

ESI-MS calcd for $C_{19}H_{18}NaO_8^+$ $[M+Na]^+$ 397.1; found 397.2.

Test Example 1: Evaluation of TRPV4 activity-inhibiting action

(1) Reagent

Human duodenum-derived cells (Hutu-80 cells) and human cervical cancer-derived cells (HeLa cells) were purchased from the American Type Culture Collection; High Pure PCR Product Purification Kit was purchased from Roche Diagnostics K.K.; pcDNA 3.1 Directional TOPO Expression Kit and DMEM/F12 medium were purchased from Invitrogen; TransIT-HeLaMONSTER Transfection Kit was purchased from Mirus Bio LLC; Detachin was purchased from Genlantis Inc.; 96-well optical bottom plate was purchased from Nunc; Calcium Kit II - fluo 4 was purchased from DOJINDO Laboratories; Hanks' balanced salt solution and fetal bovine serum were purchased from Gibco; GSK1016790a was purchased from Sigma Aldrich Co. LLC.; 5x PrimeSTAR GXL Buffer, dNTPs mixture, and PrimeSTAR GXL DNA Polymerase were purchased from Takara Bio Inc.; and dimethyl sulfoxide was purchased from FUJIFILM Wako Pure Chemical Corporation.

(2) Production of human TRPV4 gene expression vector

Polymerase chain reaction (PCR) was performed under the following conditions, using cDNA prepared by reverse transcription of the total RNA extracted from the Hutu-80 cells as a template and using a primer set consisting of the oligonucleotides represented by the following base sequences synthesized with reference to the published human TRPV4 gene sequence.

<Primer set>

Forward primer: 5'-CACCATGGCGGATTCCAGCGAAGGCCC-3' (SEQ ID NO: 1)
Reverse primer: 5'-CTAGAGCGGGGCGTCATCAGTCC-3' (SEQ ID NO: 2)

<PCR condition>

a) PCR solution composition

cDNA (Template): 15 μL
5x PrimeSTAR GXL Buffer: 10 μL
dNTPs mixture (2.5 mM): 4 μL
PrimeSTAR GXL DNA Polymerase: 1 μL
Forward Primer (10 μM): 1 μL
Reverse Primer (10 μM): 1 μL
Water: 18 μL

b) Temperature and cycle condition

95°C for 2 minutes
↓
98°C for 10 seconds, 70°C for 2 minutes, 33 cycles

The resulting PCR product was purified using High Pure PCR Product Purification Kit. A human TRPV4 gene expression vector was produced using the purified PCR product and pcDNA 3.1 Directional TOPO Expression Kit.

(3) Production of human TRPV4-expressing cells

HeLa cells were cultured using a DMEM/F12 medium containing 10% fetal bovine serum. The HeLa cells were seeded in a T-75 cell culture flask at $5 \times 10^5$ cells/flask. The cells were cultured for 3 days, were then transfected with the human TRPV4 gene expression vector (8 μg) produced in the above (2) using TransIT-HeLaMONSTER Transfection

Kit, and were cultured for 1 day.

The cells were detached with Detachin, were seeded in a 96-well optical bottom plate containing a DMEM/F12 medium containing 10% fetal bovine serum at a cell density of 1.5 $\times$ $10^4$ cells/90 μL/well, and were further cultured for 1 day.

(4) Measurement of intracellular calcium ion influx rate

The intracellular calcium ion influx rate was measured using Calcium Kit II - fluo 4. Loading buffer containing Fluo 4-AM and Hanks' balanced salt solution were mixed at 1:1 and were then added to the human TRPV4-expressing cells produced in the above (3) at 180 μL/well, followed by incubation at 37°C for 1 hour. Subsequently, the fluorescence intensity (excitation wavelength: 488 nm, fluorescence wavelength: 524 nm) was measured every 2 seconds at 37°C using a fluorescence plate reader FDSS3000 (Hamamatsu Photonics K.K.). 30 seconds after the start of the measurement, GSK1016790a as a TRPV4 agonist and the 4-O-methyl-rosmarinic acid or 3,4'-di-O-methyl-rosmarinic acid solution prepared in the above Manufacturing Example as a sample were each diluted with Hanks' balanced salt solution, and a mixture solution thereof (mixed immediately before the addition) was added at 20 μL/well. The change in fluorescence intensity was measured every 2 seconds for 300 seconds. GSK1016790a was added to give a final concentration of 3 nM.

In addition, as a positive control for TRPV4 activity inhibition, HC067047, which is a TRPV4 antagonist, was added at a final concentration of 10 μM. 4-O-Methyl-rosmarinic acid, 3,4'-di-O-methyl-rosmarinic acid, GSK1016790a, and HC067047 were all used by dissolving in dimethyl sulfoxide (DMSO).

The TRPV4 activity of a sample was calculated by the following expression, and the intracellular calcium ion influx rate due to the treatment with a TRPV4 agonist, GSK1016790a, was defined as 100%.

$$\text{Intracellular calcium ion influx rate (\%)} = [(Fmax/F0) - (FmaxC2/F0C2)]/[(FmaxC1/F0C1) - (FmaxC2/F0C2)] \times 100$$

Fmax: Maximum fluorescence intensity of the well containing GSK1016790a and a sample in the period from 0 to 300 seconds after the start of the measurement,
FmaxC1: Maximum fluorescence intensity of the well containing GSK1016790a and a solvent in the period from 0 to 300 seconds after the start of the measurement,
FmaxC2: Maximum fluorescence intensity of the well containing only a solvent in the period from 0 to 300 seconds after the start of the measurement,
F0: Fluorescence intensity of the well of Fmax immediately after the start of the measurement,
F0C1: Fluorescence intensity of the well of FmaxC1 immediately after the start of the measurement, and
F0C2: Fluorescence intensity of the well containing only a solvent immediately after the start of the measurement.

The intracellular calcium ion influx rate (each group, n = 3) when a sample was added was compared to that when GSK1016790a and a solvent (DMSO) were added and was examined by a Dunnett's test. Table 1 shows the results when 4-O-methyl-rosmarinic acid or 3,4'-di-O-methyl-rosmarinic acid was added (in the table, GSK1016790a, 4-O-methyl-rosmarinic acid, 3,4'-di-O-methyl-rosmarinic acid are abbreviated as GSK, 4-Me-RA, and 3,4'-DiMe-RA, respectively). The results are expressed as the mean value (Mean) ± standard error (S.E.).

[Table 1]

| | Intracellular $Ca^{2+}$ influx rate (%) |
|---|---|
| GSK 3 nM + DMSO 0.1% | 100.00 ± 2.29 |
| GSK 3 nM + HC067047 10 μM (Positive control) | 2.46 ± 0.14 (P<0.001) |
| GSK 3 nM + 4-Me-RA 100 μM | 92.65 ± 0.89 (N.S.) |
| GSK 3 nM + 4-Me-RA 300 μM | 82.90 ± 3.04 (P<0.01) |
| GSK 3 nM + 3,4'-DiMe-RA 100 μM | 78.81 ± 1.73 (P<0.001) |
| GSK 3 nM + 3,4'-DiMe-RA 300 μM | 71.25 ± 5.52 (P<0.001) |
| n=3, Mean ± S.E. | |

As in the case of the TRPV4 antagonist which is a positive control, 4-O-methyl-rosmarinic acid and 3,4'-di-O-methyl-rosmarinic acid significantly decreased the calcium ion influx into cells by a TRPV4 agonist in a concentration-dependent manner, as shown in Table 1.

The results above show that 4-O-methyl-rosmarinic acid and 3,4'-di-O-methyl-rosmarinic acid inhibit the TRPV4 activity. The results also show that 4-O-methyl-rosmarinic acid and 3,4'-di-O-methyl-rosmarinic acid having the action of inhibiting the TRPV4 activity are effective for preventing or ameliorating an irritable bowel syndrome or an overactive bladder.

**Claims**

1. A rosmarinic acid derivative represented by the following formula (I) or a salt thereof:

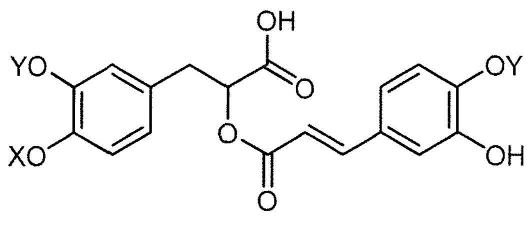

(I)

wherein, X and Y each represent a hydrogen atom or a methyl group; when X is a methyl group, Y represents a

hydrogen atom; and when X is a hydrogen atom, Y represents a methyl group.

**2.** A TRPV4 activity inhibitor comprising a rosmarinic acid derivative represented by the following formula (I) or a salt thereof as an active ingredient:

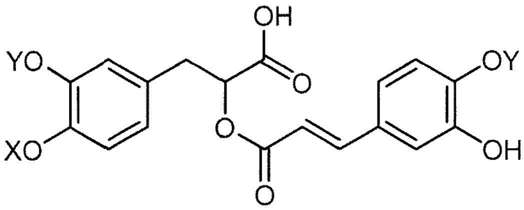

( I )

wherein, X and Y each represent a hydrogen atom or a methyl group; when X is a methyl group, Y represents a hydrogen atom; and when X is a hydrogen atom, Y represents a methyl group.

**3.** An overactive bladder-preventing or ameliorating agent comprising a rosmarinic acid derivative represented by the following formula (I) or a salt thereof as an active ingredient:

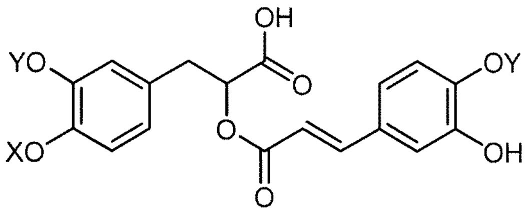

( I )

wherein, X and Y each represent a hydrogen atom or a methyl group; when X is a methyl group, Y represents a hydrogen atom; and when X is a hydrogen atom, Y represents a methyl group.

**4.** An irritable bowel syndrome-preventing or ameliorating agent comprising a rosmarinic acid derivative represented by the following formula (I) or a salt thereof as an active ingredient:

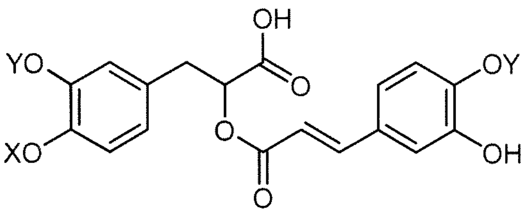

( I )

wherein, X and Y each represent a hydrogen atom or a methyl group; when X is a methyl group, Y represents a hydrogen atom; and when X is a hydrogen atom, Y represents a methyl group.

**5.** A TRPV4 activity-inhibiting food comprising a rosmarinic acid derivative represented by the following formula (I) or a salt thereof as an active ingredient:

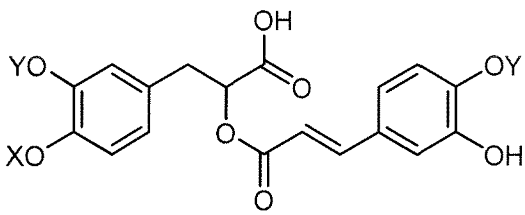

( I )

wherein, X and Y each represent a hydrogen atom or a methyl group; when X is a methyl group, Y represents a hydrogen atom; and when X is a hydrogen atom, Y represents a methyl group.

6. An overactive bladder-preventing or ameliorating food comprising a rosmarinic acid derivative represented by the following formula (I) or a salt thereof as an active ingredient:

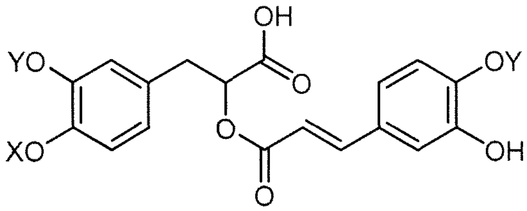

( I )

wherein, X and Y each represent a hydrogen atom or a methyl group; when X is a methyl group, Y represents a hydrogen atom; and when X is a hydrogen atom, Y represents a methyl group.

7. An irritable bowel syndrome-preventing or ameliorating food comprising a rosmarinic acid derivative represented by the following formula (I) or a salt thereof as an active ingredient:

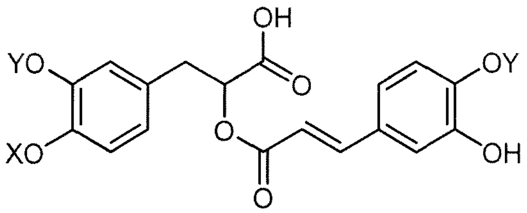

( I )

wherein, X and Y each represent a hydrogen atom or a methyl group; when X is a methyl group, Y represents a hydrogen atom; and when X is a hydrogen atom, Y represents a methyl group.

8. A use of a rosmarinic acid derivative represented by the following formula (I) or a salt thereof for manufacturing a TRPV4 activity inhibitor:

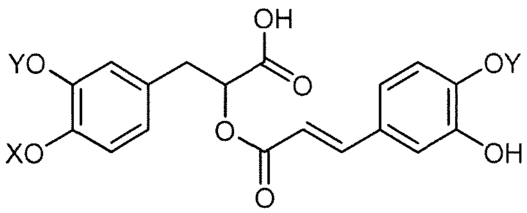

( I )

wherein, X and Y each represent a hydrogen atom or a methyl group; when X is a methyl group, Y represents a hydrogen atom; and when X is a hydrogen atom, Y represents a methyl group.

9. A use of a rosmarinic acid derivative represented by the following formula (I) or a salt thereof for manufacturing an overactive bladder-preventing or ameliorating agent:

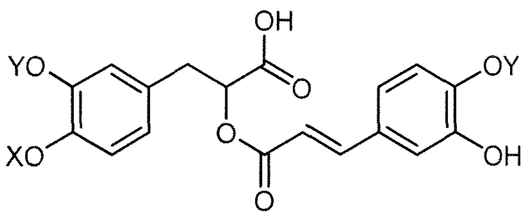

( I )

wherein, X and Y each represent a hydrogen atom or a methyl group; when X is a methyl group, Y represents a hydrogen atom; and when X is a hydrogen atom, Y represents a methyl group.

10. A use of a rosmarinic acid derivative represented by the following formula (I) or a salt thereof for manufacturing an irritable bowel syndrome-preventing or ameliorating agent:

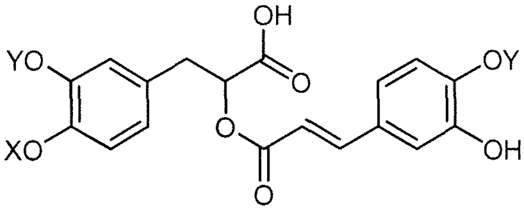

(I)

wherein, X and Y each represent a hydrogen atom or a methyl group; when X is a methyl group, Y represents a hydrogen atom; and when X is a hydrogen atom, Y represents a methyl group.

**11.** A use of a rosmarinic acid derivative represented by the following formula (I) or a salt thereof for manufacturing a TRPV4 activity-inhibiting food:

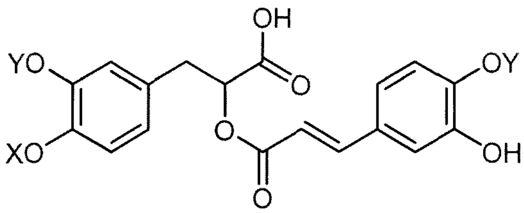

(I)

wherein, X and Y each represent a hydrogen atom or a methyl group; when X is a methyl group, Y represents a hydrogen atom; and when X is a hydrogen atom, Y represents a methyl group.

**12.** A use of a rosmarinic acid derivative represented by the following formula (I) or a salt thereof for manufacturing an overactive bladder-preventing or ameliorating food:

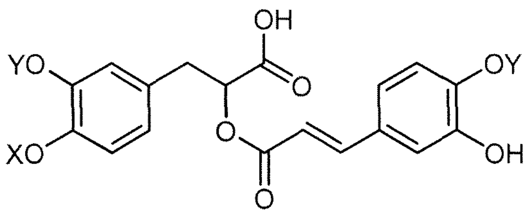

(I)

wherein, X and Y each represent a hydrogen atom or a methyl group; when X is a methyl group, Y represents a hydrogen atom; and when X is a hydrogen atom, Y represents a methyl group.

**13.** A use of a rosmarinic acid derivative represented by the following formula (I) or a salt thereof for manufacturing an irritable bowel syndrome-preventing or ameliorating food:

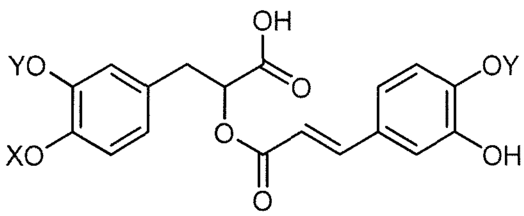

(I)

wherein, X and Y each represent a hydrogen atom or a methyl group; when X is a methyl group, Y represents a hydrogen atom; and when X is a hydrogen atom, Y represents a methyl group.

**14.** A rosmarinic acid derivative represented by the following formula (I) or a salt thereof for use in inhibiting a TRPV4 activity:

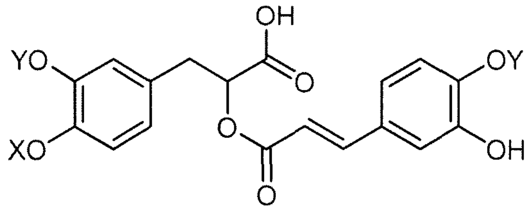

( I )

wherein, X and Y each represent a hydrogen atom or a methyl group; when X is a methyl group, Y represents a hydrogen atom; and when X is a hydrogen atom, Y represents a methyl group.

**15.** A rosmarinic acid derivative represented by the following formula (I) or a salt thereof for use in preventing or ameliorating an overactive bladder:

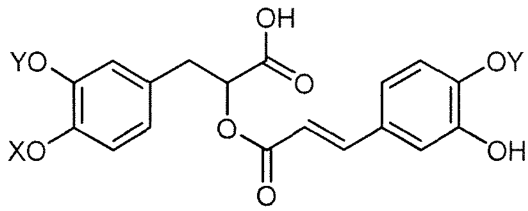

( I )

wherein, X and Y each represent a hydrogen atom or a methyl group; when X is a methyl group, Y represents a hydrogen atom; and when X is a hydrogen atom, Y represents a methyl group.

**16.** A rosmarinic acid derivative represented by the following formula (I) or a salt thereof for use in preventing or ameliorating an irritable bowel syndrome:

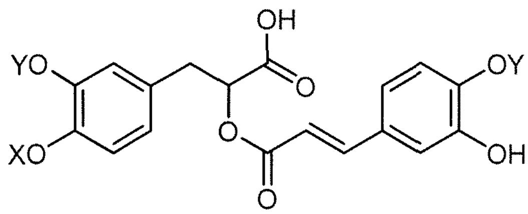

( I )

wherein, X and Y each represent a hydrogen atom or a methyl group; when X is a methyl group, Y represents a hydrogen atom; and when X is a hydrogen atom, Y represents a methyl group.

**17.** A nontherapeutic use of a rosmarinic acid derivative represented by the following formula (I) or a salt thereof for inhibiting a TRPV4 activity:

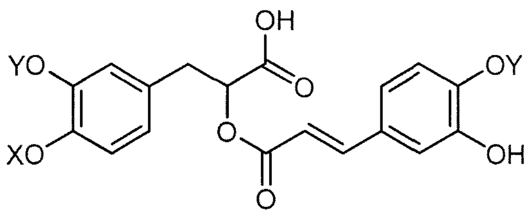

( I )

wherein, X and Y each represent a hydrogen atom or a methyl group; when X is a methyl group, Y represents a hydrogen atom; and when X is a hydrogen atom, Y represents a methyl group.

**18.** A nontherapeutic use of a rosmarinic acid derivative represented by the following formula (I) or a salt thereof for preventing or ameliorating an overactive bladder:

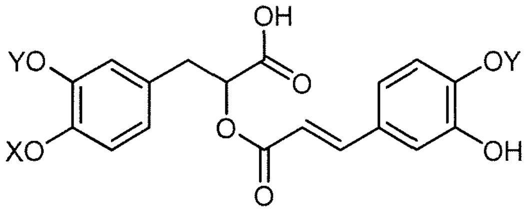

(I)

wherein, X and Y each represent a hydrogen atom or a methyl group; when X is a methyl group, Y represents a hydrogen atom; and when X is a hydrogen atom, Y represents a methyl group.

**19.** A nontherapeutic use of a rosmarinic acid derivative represented by the following formula (I) or a salt thereof for preventing or ameliorating an irritable bowel syndrome:

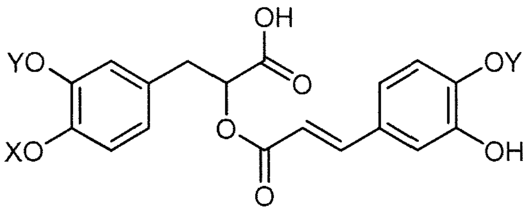

(I)

wherein, X and Y each represent a hydrogen atom or a methyl group; when X is a methyl group, Y represents a hydrogen atom; and when X is a hydrogen atom, Y represents a methyl group.

**20.** A method for inhibiting a TRPV4 activity, comprising administering to a subject in need thereof, or allowing a subject in need thereof to ingest, an effective amount of a rosmarinic acid derivative represented by the following formula (I) or a salt thereof:

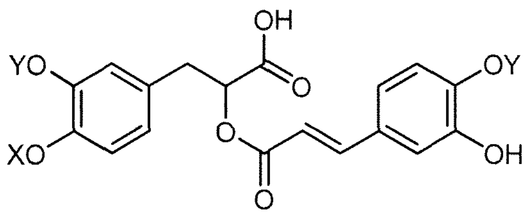

(I)

wherein, X and Y each represent a hydrogen atom or a methyl group; when X is a methyl group, Y represents a hydrogen atom; and when X is a hydrogen atom, Y represents a methyl group.

**21.** A method for preventing or ameliorating an overactive bladder, comprising administering to a subject in need thereof, or allowing a subject in need thereof to ingest, an effective amount of a rosmarinic acid derivative represented by the following formula (I) or a salt thereof:

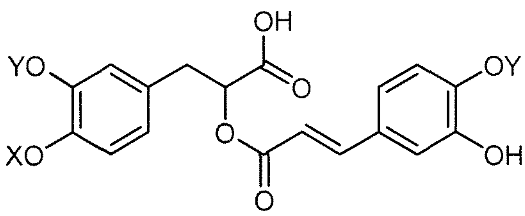

(I)

wherein, X and Y each represent a hydrogen atom or a methyl group; when X is a methyl group, Y represents a hydrogen atom; and when X is a hydrogen atom, Y represents a methyl group.

**22.** A method for preventing or ameliorating an irritable bowel syndrome, comprising administering to a subject in need thereof, or allowing a subject in need thereof to ingest, an effective amount of a rosmarinic acid derivative represented by the following formula (I) or a salt thereof:

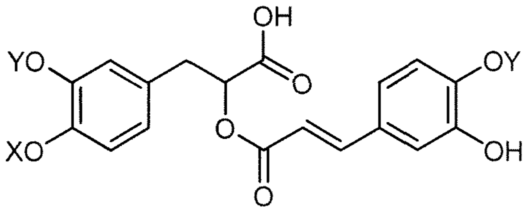

(I)

wherein, X and Y each represent a hydrogen atom or a methyl group; when X is a methyl group, Y represents a hydrogen atom; and when X is a hydrogen atom, Y represents a methyl group.

## INTERNATIONAL SEARCH REPORT

International application No.
PCT/JP2019/024322

A. CLASSIFICATION OF SUBJECT MATTER
Int.Cl. C07C69/732(2006.01)i, A23L33/10(2016.01)i, A61K31/216(2006.01)i, A61P1/12(2006.01)i, A61P13/10(2006.01)i, A61P43/00(2006.01)i, C07C69/736(2006.01)i, C07K14/47(2006.01)i
According to International Patent Classification (IPC) or to both national classification and IPC

B. FIELDS SEARCHED
Minimum documentation searched (classification system followed by classification symbols)
Int.Cl. C07C69/732, A23L33/10, A61K31/216, A61P1/12, A61P13/10, A61P43/00, C07C69/736, C07K14/47

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580(JDreamIII), CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y<br><br><br>A | JP 2005-272362 A (MEIJI SEIKA KAISHA LTD.) 06 October 2005, claims<br>(Family: none) | 1, 14-16<br>3, 4, 6, 7, 9, 10, 12, 13, 18, 19, 21, 22<br>2, 5, 8, 11, 17, 20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14.08.2019 | 27.08.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2019/024322

C (Continuation). DOCUMENTS CONSIDERED TO BE RELEVANT

| Category* | Citation of document, with indication, where appropriate, of the relevant passages | Relevant to claim No. |
|---|---|---|
| Y<br><br>A | JP 2005-526040 A (PHARMACIA CORPORATION) 02 September 2005, claims, paragraphs [0005], [0006], [0009], [0090] & US 2003/0191172 A1, claims, paragraphs [0006], [0007], [0010], [0093] & WO 2003/070233 A1 & EP 1915992 A1 & CA 2475374 A & CN 1633283 A & KR 10-2005-0005410 A | 3, 6, 9, 12, 18, 21<br>1, 2, 4, 5, 7, 8, 10, 11, 13-17, 19, 20, 22 |
| Y<br><br>A | JP 2003-521511 A (GLAXO GROUP LTD.) 15 July 2003, claims, paragraph [0004] & US 2003/0013717 A1, claims, paragraph [0005] & WO 2001/056555 A2 | 4, 7, 10, 13, 19, 22<br>1-3, 5, 6, 8, 9, 11, 12, 14-18, 20, 21 |
| A | JP 2000-72685 A (NAGASE AND CO., LTD.) 07 March 2000, claims (Family: none) | 1-22 |
| A | WO 2002/062365 A1 (MEIJI SEIKA KAISHA LTD.) 15 August 2002, claims & JP 4441177 B2 | 1-22 |
| A | JP 2013-216592 A (KAO CORPORATION) 24 October 2013, claims (Family: none) | 1-22 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014024809 A
- JP 2005139136 A
- KR 2013087877


### Non-patent literature cited in the description


- **TIAN W.** *Am. J. Physiol. Renal. Physiol.,* 2004, vol. 287, F17-F24
- **NILIUS B.** *Physiol. Rev.,* 2007, vol. 87, 165-217
- **GEVAERT T.** *J. Clin. Invest.,* 2007, vol. 117, 3453-3462
- **NICOLAS CENAC et al.** *Gastroenterology,* 2015, vol. 149, 433-444

SEQUENCE LISTING

```
<110>   Kao Corporation

<120>   ROSMARINIC ACID DERIVATIVE OR SALT THEREOF

<130>   KS1626

<150>   JP 2018-117368
<151>   2018-6-20

<160>   2

<170>   PatentIn version 3.5

<210>   1
<211>   27
<212>   DNA
<213>   Artificial Sequence

<220>
<223>    Synthetic oligonucleotide

<400>   1
caccatggcg gattccagcg aaggccc                                      27


<210>   2
<211>   23
<212>   DNA
<213>   Artificial Sequence

<220>
<223>    Synthetic oligonucleotide

<400>   2
ctagagcggg gcgtcatcag tcc                                          23
```